# EUROPEAN PATENT APPLICATION

(11) **EP 4 324 907 A1**
(43) Date of publication of application: **21.02.2024**
(21) Application number: 22788196.8
(22) Date of filing: 14.04.2022
(51) Int. Cl.: C12M 1/10, C12N 7/02

(54) **METHOD FOR PRODUCING VIRUS PARTICLES**

(30) Priority: 15.04.2021 US 202163175296 P
(71) Applicant: The University of Tokyo, Bunkyo-ku, Tokyo 113-8654 (JP)
(72) Inventor: OKADA, Takashi, Tokyo 113-8654 (JP); WADA, Mikako, Tokyo 113-8654 (JP)
(74) Representative: Vossius & Partner Patentanwälte Rechtsanwälte mbB
(86) International application number: PCT/JP2022/017762
(87) International publication number: WO 2022/220273

(57) **Abstract**

It is an object of the present invention to provide a method for producing a virus to obtain high-purity virus particles, said method being more efficient than conventional methods. Specifically, the present invention relates to a method for producing full-genome virus particles, comprising steps of purifying full-genome virus particles from a virus particle mixed solution containing empty virus particles, intermediate virus particles and the full-genome virus particles, wherein the method comprises: a step (a) of rotating a zonal rotator at a low speed and arranging the virus particle mixed solution, a liquid having a lower density than the full-genome virus particles (liquid L), and a liquid having a higher density than the liquid L (liquid H1) in this order from the rotation axis side of the rotor toward the outside; a step (b) of operating the zonal rotor after the step (a) at an ultracentrifugation mode, so as to separate the empty virus particles, the intermediate virus particles and the full-genome virus particles from one another; and a step (c) of removing the contents of the zonal rotor after the step (b), while fractionating the contents, so as to recover a fraction comprising the full-genome virus particles.

## Description

### Technical Field

The present invention relates to a method for purifying or producing virus particles. More specifically, the present invention relates to a method for purifying or producing a large amount of virus particles.

### Background Art

Gene therapy, in which genes or gene-introduced cells are administered into a human body for the purpose of treating diseases, is one of important therapeutic methods for the treatment of intractable diseases. Currently, biological methods using viral vectors are most common as methods of introducing genes into mammalian cells for the purpose of gene therapy. Viral vector is a carrier for efficiently introducing a gene into a cell and allowing the gene to express therein by incorporating the gene to be introduced for therapeutic purposes into a viral strain that has lost or has partially lost its ability to replicate and proliferate. Known viruses as origins of such viral vectors include enveloped viruses (viruses having envelopes) such as retrovirus, lentivirus, herpes virus and Sendai virus, and non-enveloped viruses (viruses that do not have envelopes) such as adenovirus and adeno-associated virus (AAV). Among others, AAV has been used in gene therapy for the treatment of various diseases because, for example, this virus is able to infect many types of cells, that this virus has no pathogenicity to humans, and that the virus particles are physically stable.

Since AAV vector is capable of efficient gene introduction for allowing a gene of interest to stably express in a cell, the AAV vector has been used so far as a means of gene delivery to target cells. Recently, clinical trials regarding gene therapy using AAV vectors have been conducted for various genetic diseases such as Parkinson's disease, cystic fibrosis, rheumatoid arthritis, lipoprotein lipase deficiency, alpha 1 antitrypsin deficiency, Duchenne muscular dystrophy (DMD), Leber's congenital amaurosis, chloridemia, hemophilia A, and hemophilia B. In order to carry out gene therapy using such AAV vectors safely and effectively, it is extremely important to overcome immune responses to the AAV vectors (Non-Patent Literature 1).

There have been various reports regarding gene therapy using AAV vectors so far. For example, in the gene therapy performed on hemophilia A and hemophilia B using AAV2, AAV8, and AAV10 vectors, it has been reported that an increase in liver enzyme activity and AAV capsid-specific T cell activation were detected, and further that the activity of Factor VIII and Factor IX was decreased (Non-Patent Literature 2 and Non-Patent Literature 3). On the other hand, it has also been reported that when gene therapy was performed on hemophilia using an AAV5 vector at a low dose, an increase in liver enzyme activity was not observed, T cell activation was suppressed to the minimum, and blood coagulation factors were maintained in most patients (Non-Patent Literature 1 and Non-Patent Literature 4).

These reports have suggested that the cellular immune response to AAV capsids induces destruction of hepatocytes into which AAV vectors have been introduced. Therefore, upon systemic administration of AAV vectors, it is considered important for safe gene therapy to use vectors with high transduction efficiency and to reduce the applied dose thereof.

To date, as methods for purifying AAV vectors, there have been reported chromatography using ion exchange columns or affinity purification columns, a method comprising a filtration step using Tangential Flow Filtration (TFF), a method of combining a sucrose density-gradient centrifugation method (Patent Literature 1) or a cesium chloride equilibrium density-gradient centrifugation method (Patent Literature 2) with the aforementioned chromatography and TFF steps, and other methods. These methods exhibit certain effects in terms of removing impurities from an AAV vector fraction. However, according to the method involving ion exchange chromatography, empty particles (AAV particles that do not contain the genome) and intermediate particles (AAV particles that do not contain the full-length genome but contain fragments of the genome) cannot be completely removed. Moreover, the method involving the combination of ion exchange chromatography with density-gradient centrifugation is effective for the removal of impurities and separation of full-genome particles (AAV particles comprising the full-length genome) that do not contain empty particles and intermediate particles, but the purification process of such full-genome particles is complicated. Furthermore, in the case of the previously reported density-gradient centrifugation, the centrifugation process requires a long period of time, and in particular, in the case of using an equilibrium density-gradient centrifugation method with cesium chloride, this method has been problematic in that the transduction rate of the AAV vector decreases if the AAV vector is in contact with cesium chloride for a long period of time (Non-Patent Literature 5 and Non Patent Literature 6). Further, the conventional ultracentrifugal purification method is limited in terms of the amount of a sample that can be handled at one time, and thus, this method has been problematic in terms of mass purification. Recently, since a starting material of a purified AAV vector has shifted from cell pellets to culture supernatants due to the progress of marketization and changes in the serotypes used, a purification method capable of handling a large amount of starting material has been required.

Taking into consideration the current situation of AAV vectors as described above, it is necessary to develop a novel method for preparing a large amount of vectors having high safety and high transduction rates.

### Citation List

### Patent Literature

Patent Literature 1: WO2018/128688
Patent Literature 2: WO2019/094253

### Non-Patent Literature

Non-Patent Literature 1: Muhuri et al., J Clin Invest. 131: e143780 doi:10.1172/JCI143780. 2021.
Non-Patent Literature 2: High et al., N Engl J Med. 381: 455-464, 2019.
Non-Patent Literature 3: Batty et al., Hemasphere 5: e540, 2021.
Non-Patent Literature 4: Rangarajan et al., N Engl J Med. 377: 2519-2530, 2017.
Non-Patent Literature 5: Hinderer et al., Hum Gene Ther. 29: 285-298, 2018.
Non-Patent Literature 6: Auricchio et al., Hum Gene Ther. 12: 71-76, 2001.

### Summary of Invention

### Technical Problem

Considering the aforementioned circumstances, it is an object of the present invention to provide a method of separating a larger amount of higher-purity virus particles in a shorter time than conventional methods.

### Solution to Problem

To purify full-genome AAV particles that maintain high purity and high transduction rate, the present inventors have investigated conditions for preparing density gradients in a cesium chloride equilibrium density-gradient centrifugation method and conditions such as centrifugation time. Specifically, the present inventors have loaded a culture supernatant containing AAV particles into a zonal rotor in which cesium chloride solutions having two different concentrations were disposed and have then performed ultracentrifugation. As a result, the present inventors have succeeded in separating/purifying full-genome particles that maintained high purity and high transduction rate from a larger volume of sample (i.e., a starting sample containing AAV) than before in a short centrifugation time (about 4 to 5 hours).

Specifically, the present invention includes the following (1) to (11).
(1) A method for producing full-genome virus particles, comprising steps of purifying full-genome virus particles from a virus particle mixed solution containing empty virus particles, intermediate virus particles and the full-genome virus particles, wherein
   the method comprises:
   a step (a) of rotating a zonal rotator at a low speed and arranging the virus particle mixed solution, a liquid having a lower density than the full-genome virus particles (liquid L), and a liquid having a higher density than the liquid L (liquid H1) in this order from the rotation axis side of the rotor toward the outside.
   a step (b) of operating the zonal rotor after the step (a) at an ultracentrifugation mode, to separate the empty virus particles, the intermediate virus particles and the full-genome virus particles from one another; and
   a step (c) of removing the contents of the zonal rotor after the step (b), while fractionating the contents, to recover a fraction comprising the full-genome virus particles.
(2) The production method according to the above (1), wherein the step (a) is a step of rotating a zonal rotator at a low speed and arranging a liquid having a lower density than the liquid L and the liquid H1 (liquid B), the virus particle mixed solution, the liquid L, and the liquid H1 in this order from the rotation axis side of the rotor toward the outside.
(3) The production method according to the above (1) or (2), wherein, in the step (c), the zonal rotor after the step (b) is rotated at a low speed, and a liquid having a further higher density than the liquid H1 (liquid H2) is introduced into the zonal rotator from the outside of the diameter direction of the zonal rotator, so that the contents of the zonal rotator are successively pushed out, and are removed from the rotation axis side of the zonal rotor, while fractionating the contents.
(4) The production method according to the above (3), wherein the liquid L, the liquid H1 and the liquid H2 comprise cesium chloride (CAS No.: 7647-17-8), iodixanol (CAS No.: 92339-11-2), iohexol (CAS No.: 66108-95-0), amidotrizoic acid (CAS No.: 737-31-5), or metrizamide (CAS No.: 31112-62-6).
(5) The production method according to the above (1), wherein the virus particles are adeno-associated virus particles, and the density of the liquid L is 1.21 to 1.38 g/mL.
(6) The production method according to the above (1), wherein the virus particles are adeno-associated virus particles and the density of the liquid H1 is 1.39 g/mL or more.
(7) A zonal rotator, in which a virus particle mixed solution containing empty virus particles, intermediate virus particles and full-genome virus particles, a liquid having a lower density than the full-genome virus particles (liquid L), and a liquid having a higher density than the liquid L (liquid H1) are arranged in this order from the rotation axis side of the rotor toward the outside.
(8) The zonal rotor according to the above (7), wherein a liquid having a lower density than the liquid L and the liquid H1 (liquid B) is arranged closer to the rotation axis side of the rotor than the virus particle mixed solution.
(9) The zonal rotor according to the above (7) or (8), wherein the liquid L, the liquid H1 and the liquid H2 comprise cesium chloride (CAS No.: 7647-17-8), iodixanol (CAS No.: 92339-11-2), iohexol (CAS No.: 66108-95-0), amidotrizoic acid (CAS No.: 737-31-5), or metrizamide (CAS No.: 31112-62-6).
(10) The zonal rotor according to the above (7), wherein the virus particles are adeno-associated virus particles, and the density of the liquid L is 1.21 to 1.38 g/mL.
(11) The zonal rotor according to the above (7), wherein the virus particles are adeno-associated virus particles and the density of the liquid H1 is 1.39 g/mL or more.

It is to be noted that the preposition "to" sandwiched between numerical values is used in the present description to mean a numerical value range including the numerical values located left and right of the preposition.

### Advantageous Effects of Invention

According to the present invention, a method for producing full-genome virus particles having high transduction rate is provided. As a result, it becomes possible to improve the safety and effectiveness of gene therapy using a virus vector.

### Brief Description of Drawings

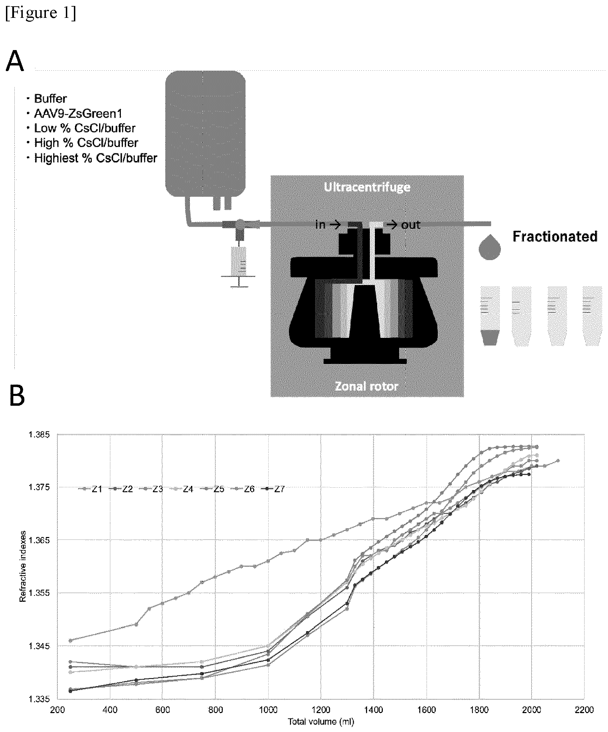

[Figure 1] Figure 1 shows separation of adeno-associated virus (AAV) vectors by cesium chloride equilibrium density-gradient ultracentrifugation using a zonal rotor.
Figure 1A shows an outline of equilibrium density-gradient ultracentrifugation with cesium chloride, in which a zonal rotor is used. Figure 1B shows the refractive index (RI) of each fraction obtained by fractionation of the solution in the rotor after completion of the density-gradient ultracentrifugation. In Z 1, density-gradient ultracentrifugation was carried out with a density gradient produced using cesium chloride solutions having 4 levels of concentrations (15%, 25%, 33%, and 40%), and the refractive index of each fraction was then measured. In Z2 to Z7, density-gradient ultracentrifugation was carried out with a density gradient produced using cesium chloride solutions having 2 levels of concentrations (25% to 27% and 40%), and the refractive index of each fraction was then measured.

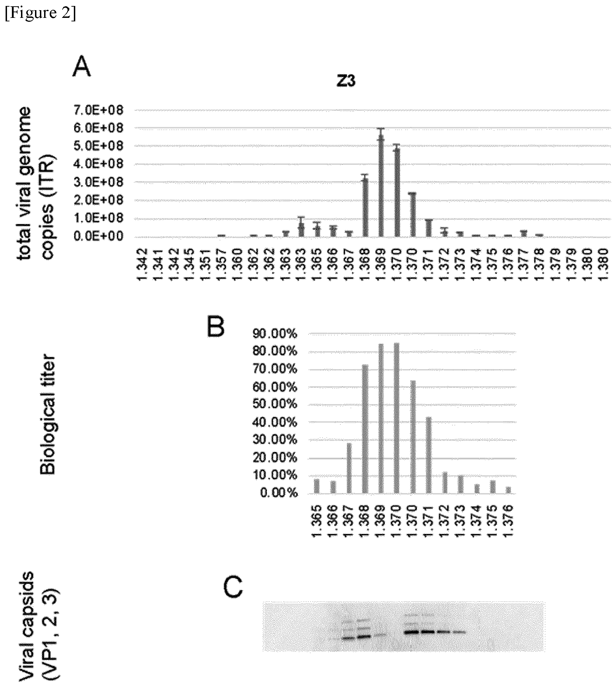

[Figure 2] Figure 2 shows the results obtained by performing calculation of the genome copy number, evaluation of the transduction rate, and detection of capsid proteins for the fractions comprising the separated full-genome virus particles (particles containing the full-length genome), intermediate virus particles (particles containing fragments of the genome), and empty virus particles (particles that do not contain the genome). Figure 2A shows the results obtained by examining the AAV genome copy number according to quantitative polymerase chain reaction (qPCR) using primers to ITR (inverted terminal repeat). Figure 2B shows the results obtained by evaluating the transduction rate of AAV using the expression of ZsGreen1 introduced into 293EB cells. Figure 2C shows the results obtained by detecting AAV capsids according to Western blotting using anti-VP1, VP2 and VP3 antibodies.

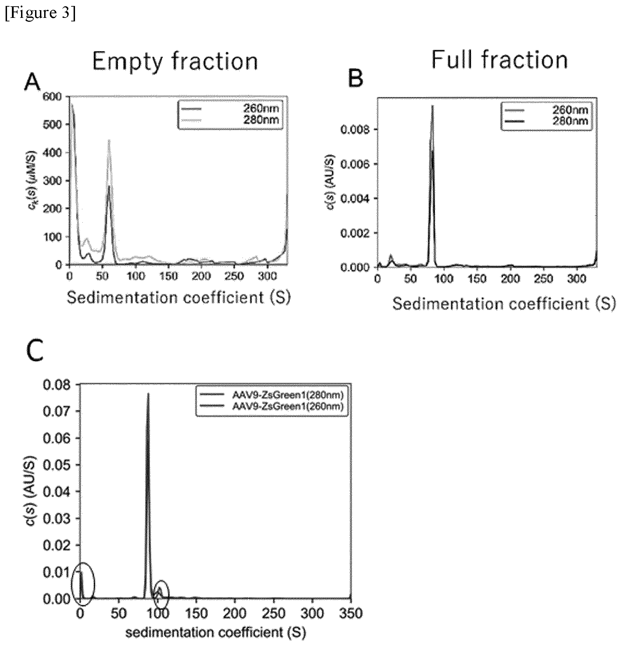

[Figure 3] Figure 3 shows the results obtained by comparing AAV vector particles purified from a culture supernatant and AAV vector particles purified from a cell lysate in terms of purity. According to analytical ultracentrifugation, the peak (A) of empty virus particles and the peak (B) of full-genome virus particles were completely separated from each other and were detected. In addition, in the AAV vector fraction purified from the cell lysate with a zonal rotor, other peaks (peaks enclosed with the lines) other than the AAV vector particles were observed (C).

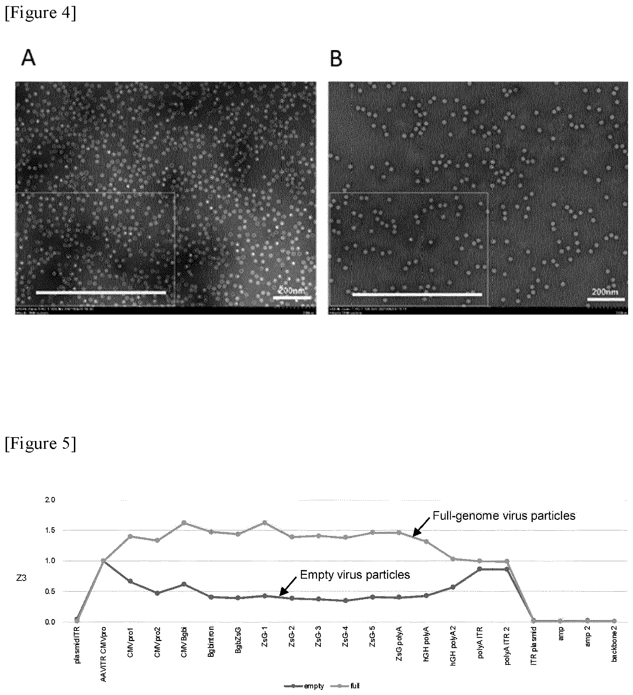

[Figure 4] Figure 4 shows the results obtained by observing full-genome AAV particles and intermediate AAV virus particles by transmission electron microscopy. Figure 4A shows the results of observation of an empty AAV particle fraction, and Figure 4B shows the results of observation of a full-genome AAV particle fraction.
[Figure 5] Figure 5 shows the results obtained by analyzing AAV virus particles according to droplet digital PCR (ddPCR). Various primer sets were designed to cover the whole region of the AAV genome. The genome regions packaged in full-genome AAV particles, intermediate AAV particles and empty AAV particles were evaluated according to ddPCR. The results of Experiment ID: Z3 are shown.

### Description of Embodiments

Hereafter, the embodiments for carrying out the present invention will be described.

A first embodiment relates to a method for producing full-genome virus particles, the method comprising using solutions having two different concentrations (densities) to form a density gradient in a zonal rotor by solutes in the solutions, and then separating and purifying full-genome virus particles from empty virus particles and intermediate virus particles based on the density gradient.

More specifically, the first embodiment is a method for producing full-genome virus particles, comprising steps of purifying full-genome virus particles from a virus particle mixed solution containing empty virus particles, intermediate virus particles, and the full-genome virus particles; wherein the method comprises:
a step (a) of rotating a zonal rotator at a low speed and arranging the virus particle mixed solution, a liquid having a lower density than the full-genome virus particles (liquid L), and a liquid having a higher density than the liquid L (liquid H1) in this order from the rotation axis side of the rotor toward the outside.
a step (b) of operating the zonal rotor after the step (a) at an ultracentrifugation mode, to separate the empty virus particles, the intermediate virus particles and the full-genome virus particles from one another; and
a step (c) of removing the contents of the zonal rotor after the step (b), while fractionating the contents, to recover a fraction comprising the full-genome virus particles.

Moreover, the step (a) may optionally be a step of rotating a zonal rotator at a low speed and arranging a liquid having a lower density than the liquid L and the liquid H1 (liquid B), the virus particle mixed solution, the liquid L, and the liquid H1, from the rotation axis side of the rotor toward the outside in this order, namely, in the order of the liquid B, the virus particle mixed solution, the liquid L, and liquid H1.

Herein, the full-genome virus particle means a virus particle, in which a full-length genome (in the case of a viral vector, a full-length vector genome comprising a foreign gene) is packaged, and which could infect target cells. The intermediate virus particle is a virus particle, in which a part of the full-length genome is packaged but the ability to infect target cells is lost or is significantly reduced. In addition, the empty virus particle means a virus particle that contains almost no genome and has almost no ability to infect target cells. To ensure the safety, etc. of virus particles used for gene therapy, such as AAV vectors, it is strongly required to prepare high-purity full-genome virus particles, and it is extremely highly necessary to separate full-genome virus particles from intermediate virus particles and empty virus particles.

In the present embodiment, step (a) is a step of injecting the virus particle mixed solution into the zonal rotor, while producing a density gradient in the zonal rotor by using a solution having a lower density than the full-genome virus particles (liquid L) and a solution having a higher density than the liquid L (liquid H1). The step (a) can be carried out, for example, by loading the virus particle mixed solution, the liquid L and the liquid H1 in this order into the zonal rotor, or by loading a liquid having a lower density than the liquid L and the liquid H (liquid B), the virus particle mixed solution, the liquid L and the liquid H1 in this order (namely, in the order of the liquid B, the virus particle mixed solution, the liquid L and the liquid H1) into the zonal rotor, while rotating the zonal rotor at a low speed (for example, 1,000 rpm to 4,000 rpm; approximately 100 x g to 1,600 x g, for approximately 30 minutes to 1 hour). After completion of the low-speed rotation operation, a linear density gradient is produced between the density of the liquid L and the density of the liquid H1 from the rotation axis side in the rotor toward the outside. The virus particles are retained on the rotation axis side in the rotor.

When the virus particles are AAV vectors, the densities of the full-genome virus particles, the intermediate virus particles, and the empty virus particles are about 1.39 g/cm³ to 1.41 g/cm³, about 1.35 g/cm³ to 1.38 g/cm³, and about 1.3 g/cm³3 to 1.35 g/cm³, respectively, although a slight margin of error may occur in all types of particles. To separate the full-genome virus particle from the intermediate virus particles and the empty virus particle, the density of the liquid L may be set to be, for example, about 1.21 g/cm³ to 1.38 g/cm³, or about 1.22 g/cm³ to 1.29 g/cm³. On the other hand, the density of the liquid H1 is desirably higher than the density of the full-genome virus particles, and the density of the liquid H1 may be, for example, about 1.39 g/cm³ or more. Besides, as the density of the liquid H1 increases, the produced density gradient becomes sharp, and as a result, the full-genome virus particles are concentrated and recovered. However, since the possibility of contamination by intermediate virus particles and the like is also increased, the density of the liquid H1 may be set to be, for example, about 1.39 g/cm³ to 1.45 g/cm³.

In the present embodiment, the ratio between the volume of the liquid L and the liquid H1 (the total volume of the liquid L and the liquid H1; the liquid L + the liquid H1) and the volume of the virus particle mixed solution (i.e. the volumes of the liquid L + the liquid H1 : the volume of the virus particle mixed solution), which are to be injected into the zonal rotor, is not particularly limited. The ratio may be, for example, about 1 : 1 to 4, about 1 : 1 to 3, or about 1 : 1 to 2. Moreover, the ratio between the volume of the liquid H1 and the volume of the liquid L, which are to be injected into the zonal rotor (i.e. the volume of the liquid H1 : the volume of the liquid L), is not particularly limited, and it may be, for example, about 1 : 1 to 3, about 1 : 1 to 2, or about 1 : 1 to 1.5.

Besides, the total volume of the virus particle mixed solution, the liquid L and the liquid H1, which are to be injected into the zonal rotor, depends on the rotor capacity of the zonal rotor used, and those skilled in the art can appropriately select an optimal injected amount.

In the present embodiment, step (b) is a step of operating the zonal rotor after the step (a) at an ultracentrifugation mode (high-speed rotation), to separate the empty virus particles, the intermediate virus particles and the full-genome virus particles from one another. The ultracentrifugation mode in the step (b) is not particularly limited, and the rotation speed may be set to be, for example, about 30,000 rpm to about 40,000 rpm (approximately 90,000 x g to 160,000 x g) for the operation. The operation time at an ultracentrifugation mode may be, for example, about 3 hours to about 10 hours, or about 4 hours to about 6 hours.

In the present embodiment, the step (c) is a step of removing the contents of the zonal rotor after the step (b), while fractionating the contents, to recover a fraction comprising the full-genome virus particles. Herein, the method of removing the contents of the zonal rotor from the rotor can be easily selected by those skilled in the art. For instance, a liquid having a further higher density than the liquid H1 (liquid H2) may be introduced into the zonal rotator from the outside of the diameter direction of the zonal rotator, so that the contents of the zonal rotator may be successively pushed out, and the contents may be removed from the rotor. The contents pushed out of the rotor are fractionated from the rotation axis side of the rotor using a fraction collector or the like at predetermined volume intervals, so that the full-genome virus particles can be separated from the intermediate virus particles and the empty virus particles. The contents of the zonal rotor can be pushed out of the rotor by rotating the rotor at a low speed (e.g., approximately 1,000 rpm to 4,000 rpm; approximately 100 x g to 1,600 x g), while injecting the liquid H2 into the zonal rotor. The contents pushed out of the rotor may be recovered from the rotation axis side of the rotor, while fractionating them using a fraction collector or the like. Herein, it may be adequate if the density of the liquid H2 is higher than the density of the liquid H1.

In the present embodiment, the virus particle mixed solution, the liquid L, the liquid H1, and the liquid H2 may comprise cesium chloride (CAS No.: 7647-17-8), iodixanol (CAS No.: 92339-11-2), iohexol (CAS No.: 66108-95-0), amidotrizoic acid (CAS No.: 737-31-5), metrizamide (CAS No.: 31112-62-6), or the like. The density gradient produced in the zonal rotor may be a density gradient formed with cesium chloride, iodixanol, iohexol, amidotrizoic acid, metrizamide or the like, and it is preferably a density gradient formed with cesium chloride. Moreover, the liquid B, the virus particle mixed solution, the liquid L, the liquid H1, and the liquid H2 may comprise buffers (e.g. a phosphate buffer, a HEPES buffer, and a Tris buffer), salts, and the like, as well as water.

The purity of the full-genome virus particles obtained after the step (c) of the present embodiment (the percentage of the full-genome virus particles in the total virus particles) is, for example, 80% or more, preferably 90% or more, and more preferably 95% or more.

The virus (particles) of the present embodiment may include, but are not limited to, wild-type viruses and foreign gene-retaining viruses that are used as vectors (viral vectors). The types of such viruses are not particularly limited, and both enveloped viruses and non-enveloped viruses are included in the viruses of the present embodiment.

The enveloped virus is a virus whose viral genome and protein shell called capsid are covered with a membrane-like structure (envelope), whereas the non-enveloped virus is a virus that does not have such an envelope. Examples of known enveloped viruses may include DNA viruses such as herpesvirus, poxvirus, and hepadnavirus, and RNA viruses such as flavivirus, togavirus, coronavirus, orthomyxovirus, paramyxovirus, rhabdovirus, bunyavirus and retrovirus. On the other hand, examples of known non-enveloped viruses may include DNA viruses such as adenovirus, adeno-associated virus (AAV), and papillomavirus, and RNA viruses such as picornavirus, calicivirus, norovirus, and rotavirus. A preferred virus used in the present embodiment is an adeno-associated virus (including an adeno-associated virus vector).

The present embodiment will be further described by taking the case of producing full-genome virus particles of adeno-associated virus vectors (AAV vectors) as an example. For example, cesium chloride or the like can be used as a density gradient medium. As a liquid L, for example, a cesium chloride solution having a density of approximately 25 wt% to 28 wt% (approximately 1.22 g/cm³ to 1.29 g/cm³) may be prepared, and as a liquid H1, for example, a cesium chloride solution having a density of approximately 40 wt% to 42 wt% (approximately 1.42 g/cm³ to 1.45 g/cm³) may be prepared, and thereafter, the thus prepared solutions may be injected into a zonal rotor (for example, P32ZT or P35ZT; both rotors have a maximum rotor lid diameter of 24 cm, a maximum inner diameter of 17.78 cm, and a maximum radius of rotation (Rmax) of 8.89 cm), Eppendorf Himac Technologies, etc.). The virus particle mixed solution, the liquid L, and the liquid H1 may be injected into the rotor in this order, while operating the rotor at a low rotation speed (for example, approximately 3,000 rpm when P32ZT or P35ZT is used). By operating the rotor at such a low rotation speed, a density gradient is formed with a cesium chloride solution from the density of virus particle mixed solution and the liquid L to the density of the liquid H1, from the rotation axis side of the rotor toward the outside (this is the step (a) of the present embodiment). Once the density gradient is formed with cesium chloride, the rotor may be operated by high-speed rotation (for example, approximately 30,000 rpm to 35,000 rpm when P32ZT or P35ZT is used) for approximately 4 to 5 hours. By operating the motor by such high-speed rotation at an ultracentrifugation mode, empty virus particles, intermediate virus particles, and full-genome virus particles are banded in this order from the rotation axis side of the rotor toward the outside (the edge side of the rotor) (this is the step (b) of the present embodiment). The contents of the rotor can be pushed out of the rotor in order from the solution close to the rotor axis side by performing low-speed rotation (for example, approximately 3,000 rpm when P32ZT or P35ZT is used), while injecting a liquid H2 having a higher density than the liquid H1 (for example, a cesium chloride solution having a density of approximately 42wt% to 45 wt% (approximately 1.45 g/cm³ to 1.49 g/cm³)) from the outside of the rotor. The solution pushed out of the rotor is recovered while fractionating it using a fraction collector or the like, so that a fraction comprising full-genome virus particles can be obtained (this is the step (c) of the present embodiment).

In the present embodiment, the virus particle mixed solution (a mixed solution containing, at least, full-genome virus particles) can be prepared by culturing virus-producing cells and then obtaining it from the virus-producing cells, a cell culture solution, etc. The virus particle mixed solution can be easily prepared by those skilled in the art according to appropriate means. In addition, the virus particle mixed solution obtained by concentrating or roughly purifying the culture solution of virus-producing cells, the lysate of the cells by TFF (Tangential Flow Filtration) or column chromatography, or the like may be used as a starting sample in the present embodiment.

In the present embodiment, the "virus-producing cells" mean cells having the ability to produce the elements necessary to form virus particles and to produce viruses. The virus-producing cells may be either artificially produced cells that can produce viruses or cells that have been infected with viruses in the natural environment and as a result, can produce such viruses. Preferably, the virus-producing cells used in the present embodiment are artificially produced virus-producing cells, and particularly preferably, the virus is a non-enveloped virus.

The method of artificially producing virus-producing cells differs depending on every virus, and the details thereof have already been described in many reviews, etc. Thus, please refer to such reviews. Herein, only an outline of the production of viral vector-producing cells will be described.

When virus particles that function as vectors are produced, a plasmid, which deletes a region encoding a non-structural protein of a virus (a protein associated with virus proliferation, etc.) and a region encoding a structural protein of a virus (a protein such as a capsid) and instead, into which a gene of interest is inserted, a plasmid encoding the non-structural protein and structural protein of the virus, a plasmid encoding other necessary genes depending on the type of the viral vector, and the like are introduced into any given cells, so that virus-producing cells can be produced.

For example, in the case of AAV vectors, a plasmid containing a gene of interest, a plasmid containing a gene encoding a Rep protein (a protein necessary for virus replication) or a Cap protein (a protein that constitutes a capsid), and a plasmid containing a gene encoding an adenovirus-derived E1a protein, E1b protein, E2 protein, E4 protein, etc. are introduced into HEK293 cells, HEK293T cells or other cells, so that AAV vector-producing cells can be produced.

The culture conditions for virus-producing cells have already been known, and those skilled in the art can select the culture conditions, as appropriate, depending on the type of virus. The culture conditions are not particularly limited, and for example, the cells may be cultured in a medium supplemented with necessary supplements (growth factors, amino acids, etc.) and serum, such as DMEM or IMDM, at a temperature of about 30°C to 38°C in a CO₂ concentration of approximately 5% to 10% for approximately several days to 20 days.

A virus-containing sample may be either an extract obtained by extracting viruses from virus-producing cells or a roughly purified product of the extract. For example, in the case of viruses released into a medium, a culture solution may be recovered after the culture of virus-producing cells and maybe then used as a sample. In the case of viruses accumulated in cells, the recovered virus-producing cells may be crushed by a freeze-thaw method or an ultrasonic crushing method, the debris and the like may be removed from the crushed cells, and the residue may be then used as a sample. Besides, since many reagents, kits, and the like for preparing virus-containing samples from virus-producing cells are commercially available, these reagents and kits, etc. may be used to prepare such virus-containing samples.

A second embodiment relates to a zonal rotator, in which a virus particle mixed solution containing empty virus particles, intermediate virus particles and full-genome virus particles, a liquid L, and a liquid H1, or a liquid B, the above-described virus particle mixed solution, the liquid L, and the liquid H1 are arranged in this order from the rotation axis side of the rotor toward the outside. The zonal rotor according to the second embodiment is the rotor in the step (a) of the first embodiment, and it is, for example, a rotor that is rotating at a low speed. In the zonal rotor according to the second embodiment, a liquid having a density that is the density of the liquid L or higher and the density of the liquid H1 or lower may be stored in a state that forms a density gradient. For the liquid B, the liquid L and the liquid H1, please refer to the description regarding the first embodiment.

When a translation of the present description includes singular terms with the articles "a," "an," and "the," these terms include not only single items but also multiple items, unless otherwise clearly specified from the context.

Hereinafter, the present invention will be further described in the following examples. However, these examples are only illustrative examples of the embodiments of the present invention, and thus, are not intended to limit the scope of the present invention.

### Examples

### 1. Methods

### 1-1. Preparation of AAV vectors

AAV vectors were prepared on a large scale from a culture supernatant (conditioned medium) and were then recovered according to a previous report (Verdera et al., Mol Ther 28: 723-746, 2020).

Using a 2 x 500 mL flask (HYPERFlask, MilliporeSigma) or a 1 L bioreactor (iCELLis Nano Bioreactor), the 293EB cell line expressing adenovirus E1a, and adenovirus E1b and Bcl-_{XL} (Tomono et al., Hum Gene Ther Methods 30: 137-143, 2019.) was cultured in Dulbecco's Modified Eagle Medium (DMEM, FUJIFILM Wako) supplemented with 10% fetal bovine serum (Thermo Fisher) for 4 days. Subsequently, AAV vector plasmids (encoding ZsGreen1 or ZsGreenl-DR) and helper plasmids were transfected into the 293EB cells in DMEM (serum-free) containing 2 mM L-glutamine, 12.1% (w/v) NaHCO₃, and 12.9% D-glucose, using polyethyleneimine hydrochloride (PEI MAX, Polysciences). Five days after the transfection, AAV vectors were recovered from a culture supernatant (810 mL) and were then treated with 18.5 U/mL endonuclease (Kaneka) in the presence of 5 mM MgCh at 37°C for 30 minutes.

### 2. Purification of full-genome AAV vectors by cesium chloride equilibrium density-gradient ultracentrifugation using zonal rotor

To the prepared AAV vectors, CsCl (FUJIFILM Wako) and an HNE buffer (50 mM 4-(2-hydroxyethyl)-1-piperazineethanesulfonic acid (HEPES) (pH 7.4), 0.15 M NaCl, and 25 mM ethylenediaminetetraacetic acid (EDTA) or an HN buffer (50 mM HEPES (pH 7.4) and 0.15 M NaCl) was added (5% CsCl) (Table 1). The cesium chloride density gradients of Experiment ID: Z2 to Z7 were each produced by injecting the following solutions into the zonal rotor (P32ZT or P35ZT, Eppendorf Himac Technologies) from the outside (edge side) of the zonal rotor in the order of (1), (2), (3), and (4), and then centrifuging the obtained solution at 3,000 rpm:
(1) 200 mL of 5%(w/v) CsCl (in the HNE or HN buffer),
(2) 900 mL to 1 L of 5%(w/v) CsCl containing AAV vectors (in the HNE or HN buffer),
(3) 300 mL of 25% to 27% CsCl (in the HNE or HN buffer), and
(4) 200 to 300 mL of 40% CsCl (in the HNE or HN buffer).

**[Table 1]**

| Experiment ID | **Z1** | **Z2** | **Z3** | **Z4** | **Z5** | **Z6** |
|---|---|---|---|---|---|---|
| Rotor | P32ZT | P32ZT | P32ZT | P32ZT | P35ZT | P35ZT |
| Number of rotations (rpm) | 30,000 | 30,000 | 30,000 | 30,000 | 35,000 | 35,000 |
| Centrifugati on time | 10h | 4h | 5h | 5h | 4h | 4h |
| Vector type | AAV9-ZsGreen1 | AAV9-ZsGreen1 | AAV9-ZsGreen1 | AAV6-ZsGreen1 -DR | AAV9-ZsGreen1 | AAV9-ZsGreen1 |
| CsCI solution/ HNE or HN buffer | HNE 200 mL | | | | | |
| | AAV (5%) 300 mL | HNE 200 mL | HNE 200 mL | HN 200 mL | HN 200 mL | HN 200 mL |
| | 15% 300 mL | AAV (5%) 900 mL | AAV (5%) 900 mL | AAV(5%) 900 mL | AAV(5%) 900 mL | AAV(5%) 1000 mL |
| | 25% 300 mL | 27% 300 mL | 25% 300 mL | 25% 300 mL | 25% 300 mL | 25% 300 mL |
| | 33% 500 mL | 40% 300 mL | 40% 300 mL | 40% 300 mL | 40% 300 mL | 40% 200 mL |
| | 40% 200 mL | | | | | |

After production of the density gradient, the zonal rotor was centrifuged at 30,000 rpm to 35,000 rpm for 4 to 10 hours (himac CP 80NX, Eppendorf Himac Technologies) to separate full-genome AAV vectors from intermediate and empty vectors. After completion of the ultracentrifugation, 42% to 45% CsCl (in the HNE or HN buffer) was slowly injected into the zonal rotor from the outside of the rotor, while the zonal rotor was centrifuged at 3,000 rpm, so that the solution inside the rotor (a cesium chloride density-gradient solution containing the AAV vectors) was pushed out of the rotor, and the extruded solution was recovered, while fractionating it using a fraction collector (Figure 1A). The refractive index of each fraction was measured using an Abbe refractometer (NAR-1T LIQUID, Atago) or a digital refractometer (RX 5000i, Atago) (Figure 1B, Table 2, and Table 3). Using a 20 kDa molecular weight cut-off dialysis cassette (#66003, Thermo Fisher), the recovered fractions were each dialyzed against 0.5 mM MgCl₂ (an aqueous solution) at 4°C for approximately 2 hours, and were then dialyzed against 0.5 mM MgCl₂ (a PBS solution) in a 20 kDa molecular weight cut-off dialysis cassette (#66003, Thermo Fisher) at 4°C overnight.

**[Table 2]**

| Fraction | Volume (mL) | Z1 |
|---|---|---|
| 1 | 250 | 1.346 |
| 2 | 250 | 1.349 |
| 3 | 50 | 1.352 |
| 4 | 50 | 1.353 |
| 5 | 50 | 1.354 |
| 6 | 50 | 1.355 |
| 7 | 50 | 1.357 |
| 8 | 50 | 1.358 |
| 9 | 50 | 1.359 |
| 10 | 50 | 1.360 |
| 11 | 50 | 1.360 |
| 12 | 50 | 1.361 |
| 13 | 50 | 1.3625 |
| 14 | 50 | 1.363 |
| 15 | 50 | 1.365 |
| 16 | 50 | 1.365 |
| 17 | 50 | 1.366 |
| 18 | 50 | 1.367 |
| 19 | 50 | 1.368 |
| 20 | 50 | 1.369 |
| 21 | 50 | 1.369 |
| 22 | 50 | 1.370 |
| 23 | 50 | 1.371 |
| 24 | 50 | 1.372 |
| 25 | 50 | 1.372 |
| 26 | 50 | 1.373 |
| 27 | 50 | 1.375 |
| 28 | 50 | 1.376 |
| 29 | 50 | 1.377 |
| 30 | 50 | 1.378 |
| 31 | 50 | 1.378 |
| 32 | 50 | 1.379 |
| 33 | 50 | 1.379 |
| 34 | 50 | 1.380 |
| 35 | 50 | 1.380 |

**[Table 3]**

| Fraction | Volume (mL) | L2 | Z3 | Z4 | Z5 |
|---|---|---|---|---|---|
| 1 | 250 | 1.341 | 1.342 | 1.340 | 1.337 |
| 2 | 250 | 1.341 | 1.341 | 1.341 | 1.338 |
| 3 | 250 | 1.341 | 1.342 | 1.342 | 1.339 |
| 4 | 250 | 1.344 | 1.345 | 1.345 | 1.343 |
| 5 | 150 | 1.351 | 1.351 | 1.351 | 1.351 |
| 6 | 150 | 1.356 | 1.357 | 1.357 | 1.357 |
| 7 | 30 | 1.359 | 1.360 | 1.359 | 1.361 |
| 8 | 30 | 1.361 | 1.362 | 1.361 | 1.362 |
| 9 | 30 | 1.362 | 1.362 | 1.362 | 1.364 |
| 10 | 30 | 1.363 | 1.363 | 1.363 | 1.365 |
| 11 | 30 | 1.364 | 1.363 | 1.364 | 1.366 |
| 12 | 30 | 1.364 | 1.365 | 1.364 | 1.367 |
| 13 | 30 | 1.365 | 1.366 | 1.365 | 1.368 |
| 14 | 30 | 1.367 | 1.367 | 1.366 | 1.369 |
| 15 | 30 | 1.367 | 1.368 | 1.367 | 1.370 |
| 16 | 30 | 1.368 | 1.369 | 1.368 | 1.371 |
| 17 | 30 | 1.369 | 1.370 | 1.368 | 1.372 |
| 18 | 30 | 1.370 | 1.370 | 1.369 | 1.374 |
| 19 | 30 | 1.370 | 1.371 | 1.370 | 1.376 |
| 20 | 30 | 1.371 | 1.372 | 1.371 | 1.377 |
| 21 | 30 | 1.372 | 1.373 | 1.372 | 1.379 |
| 22 | 30 | 1.373 | 1.374 | 1.373 | 1.380 |
| 23 | 30 | 1.374 | 1.375 | 1.374 | 1.382 |
| 24 | 30 | 1.376 | 1.376 | 1.376 | 1.382 |
| 25 | 30 | 1.376 | 1.377 | 1.377 | 1.383 |
| 26 | 30 | 1.377 | 1.378 | 1.378 | 1.383 |
| 27 | 30 | 1.378 | 1.379 | 1.379 | 1.383 |
| 28 | 30 | 1.378 | 1.379 | 1.380 | 1.383 |
| 29 | 30 | 1.379 | 1.380 | 1.381 | 1.383 |
| 30 | 20 | 1.379 | 1.380 | 1.381 | 1.383 |

### 1-3. Evaluation of genome copy number, capsid protein, and transduction efficiency of AAV vectors by quantitative PCR (qPCR), Western blotting, and flow cytometry.

After completion of the ultracentrifugation using the zonal rotor, the AAV genome copy number of the recovered each fraction was calculated employing AAVpro Titration Kit (for Real Time PCR) Ver. 2 (TaKaRa Bio) and using QuantStudio 3 Real-Time PCR System (Applied Biosystems).

The presence or absence of an AAV capsid protein in each fraction was confirmed by Western blotting. The samples were treated with NuPAGE LDS sample buffer (Thermo Fisher) and NuPAGE Reducing Agent (Thermo Fisher) and were then loaded on 4%-25% concentration gradient polyacrylamide gels (Criterion TG Precast Gels, Bio- Rad), followed by performing electrophoresis using SDS running buffer (Nacalai Tesque). After completion of the electrophoresis, the electrophoresed proteins were transferred onto PVDF membranes (Trans-Blot Turbo Midi PVDF Transfer Packs, Bio-Rad), and detection of the proteins was then carried out using anti-AAV VP1/VP2/VP3 mouse antibody (clone B1, Progen) and Amersham ECL Mouse IgG, HRP-linked whole Ab (Cytiva).

Transduction efficiency was evaluated using the percentage of ZsGreen1-positive cells in the transduced 293EB cells (% ZsGreen1). 293EB cells (1 × 10⁵ cells) were cultured overnight in a 24-well plate. After completion of the culture, the cells were transfected with each fraction sample (300 µL/well in serum-free DMEM (300 µL/well) containing 2 mM L-glutamine, 12.1% NaHCO₃, and 12.9% D-glucose. On the day after the transduction, 600 µL of the medium (same as above) was added to each well. The % ZsGreen1 was evaluated by a flow cytometric method (FACSMelody, Becton Dickinson) on the third day after the transduction. The results were analyzed using FlowJo Version 7.1 (Becton Dickinson).

### 1-4. Evaluation of full-genome particles (containing full-length genome) and empty particles by analytical ultracentrifugation (AUC)

The purity of the AAV vectors was analyzed using Proteome Lab XL-I ultracentrifuge (Beckman Coulter). 400 µL of the AAV vector samples were added to a center piece of a cell housing. Three cell housings and one counterbalance, to which the samples had been added, were set in an AUC rotor. After the temperature of the rotor had been set to be 20°C, ultracentrifugation was carried out at 20°C at 12,000 rpm, and light absorption (260 nm) and interference were then measured at 92 time points for 4 to 5 hours. The full-genome particles, and intermediate and empty particles of AAV were analyzed using Sedfit (National Institutes of Health), and were then visualized with GUSSI (UT Southwestern Medical Center).

### 1-5. Analysis of whole genomic regions packaged in AAVs by droplet digital PCR (ddPCR)

The whole genomic regions of AAV vectors in each fraction obtained after completion of the ultracentrifugation in the zonal rotor were analyzed by a ddPCR method. A 100-fold diluted sample (1.1 µL) was mixed with a 0.25 mM probe, a 0.5 mM forward primer and a 0.5 mM reverse primer (ddPCR Copy Number Assy, BioRad) (see Table 4), and droplets were then prepared using Automated Droplet Generator (BioRad). Thereafter, a PCR reaction was carried out in C1000 Touch Thermal Cycler (BioRad). Fluorescent signals from each droplet were detected by QX200 droplet reader (BioRad) using QuantaSoft software package (BioRad).

**[Table 4]**

| Probe/Primer | Region |
|---|---|
| dCNS114927258 | Plasmid backbone - 5'ITR |
| dCNS186312283 | 5'ITR - CMV promoter |
| dCNS461612055 | CMV promoter #1 |
| dCNS288475852 | CMV promoter #2 |
| dCNS979672264 | CMV promoter - Beta-globin intron |
| dCNS805699613 | Beta-globin intron #2 |
| dCNS412100847 | Beta-globin intron #1 |
| dCNS188296634 | Beta-globin intron - ZsGreen1 |
| dCNS683934997 | ZsGreen1 #1 |
| dCNS126933432 | ZsGreen1 #2 |
| dCNS121835080 | ZsGreen1 #3 |
| dCNS626366336 | ZsGreen1 #4 |
| dCNS568970821 | ZsGreen1 #5 |
| dCNS357921241 | ZsGreen1 - hGH polyA |
| dCNS864495584 | hGH polyA #1 |
| dCNS975683196 | hGH polyA #2 |
| dCNS435517654 | hGH polyA - 3'ITR #1 |
| dCNS953120020 | hGH polyA - 3'ITR #2 |
| dCNS787498924 | 3'ITR - plasmid backbone |
| dCNS227817477 | Ampicillin resistant gene #1 |
| dCNS770986825 | Ampicillin resistant gene #2 |
| dCNS442935619 | Plasmid Backbone #2 |

### 1-6. Morphological analysis of AAV vectors using transmission electron microscopy (TEM)

The AAV vectors were hydrophilized with an ion bombarder (Nisshin EM Co., type PIB-10), and thereafter, 3 µL of the hydrophilized samples were placed on a collodion membrane (Nisshin EM) and were then left at rest for 1 minute. After washing the resulting samples with 3 µL of water three times, the resulting samples were stained with phosphotungstic acid (PTA) for 10 seconds. The stained samples on the collodion membrane were observed by TEM (HT7800, Hitachi High-Tech).

### 2. Results

### 2-1. Separation of full-genome (containing full-length genome), intermediate (containing fragments of genome) and empty particles (containing no genome) of AAV vectors by cesium chloride density-gradient ultracentrifugation using zonal rotor

To establish a method of separating the full-genome particles of AAV vectors in a large amount from the intermediate particles and the empty particles thereof in a short time, a density gradient was formed with two concentrations of cesium chloride solutions using a zonal rotor, and an attempt was then made to separate the full-genome particles with an equilibrium density gradient (Figure 1A). As the contact time with cesium chloride becomes long, the efficiency of infection of the AAV vectors into cells decreases. Thus, it was necessary to shorten the centrifugation time as much as possible.

First, using 300 mL of a culture supernatant containing AAV vectors, a density gradient was produced with cesium chloride having four concentrations (15 wt%, 25 wt%, 33 wt% and 40 wt%). After centrifugation had been carried out at an ultracentrifugation mode (35,000 rpm) for 10 hours (Table 1), a nearly linear density gradient of cesium chloride was formed (Figure 1B and Table 2). AAV capsid proteins were detected in fractions 17 and 25. In contrast, the transduction activity of the AAV genome and ZsGreen1 was detected in fraction 25 but was not detected in fraction 17. These results show that the full-genome particles having transduction ability (fraction 25) and the empty particles that did not have such transduction ability (fraction 17) were separated from each other. It is considered that the intermediate particles were contained in the fractions between fraction 17 (the empty particles) and fraction 25 (the full-genome particles).

Even according to the cesium chloride density-gradient ultracentrifugation using cesium chloride solutions having four concentrations, it was possible to separate the full-genome particles, the intermediate particles, and the empty particles from one another. However, it is considered that the biological activity (transduction ability) of the AAV vectors would be decreased by the exposure of the AAV vectors to cesium chloride for such a long period of time as 10 hours. In view of the foregoing, it was conceived that the formation of a density gradient formed with cesium chloride solutions having two concentrations would enable the formation of a steep density gradient capable of concentrating the full-genome AAV particles within a narrow density gradient range and would also enable a reduction in the centrifugation time. Furthermore, by reducing the amount of the cesium chloride solution injected into the zonal rotor, it is possible to increase the amount of samples containing AAV vectors (Table 1).

Hence, cesium chloride solutions having two concentrations (25-27 wt% and 40 wt%, Experiment ID1: Z2 to Z5) were injected into the rotor to form a density gradient, and ultracentrifugation was then carried out. As a result, a larger volume of AAV vectors (900 to 1,000 mL) could be treated by centrifugation in a shorter time (4 to 5 hours), compared with the centrifugation using the cesium chloride solutions having four concentrations. The refractive index (density gradient) increased more sharply within a narrow range in the zonal rotor (Figure 1 and Table 3). The peaks of the AAV genome copy number (Figure 2A) and the transduction efficiency (Figure 2B) were detected in fractions 16 and 17 (RI values: 1.369 and 1.370), and the peaks of capsid proteins (Figure 2C) were also detected in these fractions. These results show that the full-genome particles were recovered in fractions 16 to 17. On the other hand, fractions 11 to 12, in which capsid proteins and a small number of genome copies were detected but ZsGreen1 transformation activity was not detected, were considered to contain the intermediate particles or the empty particles (Figures 2A, B and C, RI values: 1.365 and 1.366).

The results show that the density gradient formed with the cesium chloride solutions having two concentrations is able to separate the full-genome particles from the intermediate particles and the empty particles in a short time (4 to 5 hours), and that it is possible to treat a large amount of virus particle mixture samples.

### 2-2. Detection of high-purity full-genome particles by analytical ultracentrifugation (AUC)

Analytical ultracentrifugation was carried out to evaluate the purities of the full-genome AAV particle fraction and the empty particle fraction, which had been separated from one another according to the density gradient formed with the cesium chloride solutions having two concentrations using a zonal rotor. As a result, the full-genome particles (80S) (Figure 3B) and the empty particles (60S) (Figure 3A) having different sedimentation coefficients were each detected as a single peak. Interestingly, a full-genome particle fraction with higher purity could be separated by purification from a medium containing the AAV vectors, than by purification from a cell lysate containing the AAV vectors (compare Figures 3B and 3C).

Moreover, the full-genome AAV particles and the empty AAV particles, which had been separated from one another by cesium chloride density-gradient ultracentrifugation, were stained with phosphotungstic acid, and thereafter, their morphological characteristics were observed using a transmission electron microscope. The full-genome AAV particles were observed as hexagonal particles with white interior (Figure 4B), whereas the empty AAV particles were observed as hexagonal particles with a black dot in the center (Figure 4A). From the results of the electron microscopic observations, it is suggested that the empty AAV particles were partially compressed and stained with phosphotungstic acid more strongly.

The results show that a large amount of full-genome AAV particles were separated with high purity according to the density gradient formed with the cesium chloride solutions having two concentrations using a zonal rotor.

### 2-3. Analysis of DNAs packaged in AAV particles by droplet digital PCR (ddPCR)

To analyze DNAs packaged in the full-genome AAV particles, the intermediate AAV particles, and the empty AAV particles, 22 probes/primers were designed to detect the whole AAV genome region and the plasmid backbone region, and thereafter, evaluation was carried out by ddPCR. The full-length genome (full genome) AAV particles were confirmed to contain the whole AAV genome region and some ITR regions (Figure 5). On the other hand, short ITRs were detected in the intermediate particles and the empty particles. These results suggest that short DNA fragments containing only ITRs were generated during the process of producing AAV vectors and were then packaged in capsids.

### Industrial Applicability

The present invention provides a method for efficiently preparing virus (particles) such as viral vectors with high purity. Therefore, it is expected that the present invention will be utilized in medical fields such as gene therapy.

## Claims

1. A method for producing full-genome virus particles, comprising steps of purifying full-genome virus particles from a virus particle mixed solution containing empty virus particles, intermediate virus particles and the full-genome virus particles, wherein the method comprises:
a step (a) of rotating a zonal rotator at a low speed and arranging the virus particle mixed solution, a liquid having a lower density than the full-genome virus particles (liquid L), and a liquid having a higher density than the liquid L (liquid H1) in this order from the rotation axis side of the rotor toward the outside.
a step (b) of operating the zonal rotor after the step (a) at an ultracentrifugation mode, to separate the empty virus particles, the intermediate virus particles and the full-genome virus particles from one another; and
a step (c) of removing the contents of the zonal rotor after the step (b), while fractionating the contents, to recover a fraction comprising the full-genome virus particles.

2. The production method according to claim 1, wherein the step (a) is a step of rotating a zonal rotator at a low speed and arranging a liquid having a lower density than the liquid L and the liquid H1 (liquid B), the virus particle mixed solution, the liquid L, and the liquid H1 in this order from the rotation axis side of the rotor toward the outside.

3. The production method according to claim 1 or claim 2, wherein, in the step (c), the zonal rotor after the step (b) is rotated at a low speed, and a liquid having a further higher density than the liquid H1 (liquid H2) is introduced into the zonal rotator from the outside of the diameter direction of the zonal rotator, so that the contents of the zonal rotator are successively pushed out, and are removed from the rotation axis side of the zonal rotor, while fractionating the contents.

4. The production method according to claim 3, wherein the liquid L, the liquid H1 and the liquid H2 comprise cesium chloride (CAS No.: 7647-17-8), iodixanol (CAS No.: 92339-11-2), iohexol (CAS No.: 66108-95-0), amidotrizoic acid (CAS No.: 737-31-5), or metrizamide (CAS No.: 31112-62-6).

5. The production method according to claim 1, wherein the virus particles are adeno-associated virus particles, and the density of the liquid L is 1.21 to 1.38 g/mL.

6. The production method according to claim 1, wherein the virus particles are adeno-associated virus particles and the density of the liquid H1 is 1.39 g/mL or more.

7. A zonal rotator, in which a virus particle mixed solution containing empty virus particles, intermediate virus particles and full-genome virus particles, a liquid having a lower density than the full-genome virus particles (liquid L), and a liquid having a higher density than the liquid L (liquid H1) are arranged in this order from the rotation axis side of the rotor toward the outside.

8. The zonal rotor according to claim 7, wherein a liquid having a lower density than the liquid L and the liquid H1 (liquid B) is arranged closer to the rotation axis side of the rotor than the virus particle mixed solution.

9. The zonal rotor according to claim 7 or claim 8, wherein the liquid L, the liquid H1 and the liquid H2 comprise cesium chloride (CAS No.: 7647-17-8), iodixanol (CAS No.: 92339-11-2), iohexol (CAS No.: 66108-95-0), amidotrizoic acid (CAS No.: 737-31-5), or metrizamide (CAS No.: 31112-62-6).

10. The zonal rotor according to claim 7, wherein the virus particles are adeno-associated virus particles, and the density of the liquid L is 1.21 to 1.38 g/mL.

11. The zonal rotor according to claim 7, wherein the virus particles are adeno-associated virus particles and the density of the liquid H1 is 1.39 g/mL or more.
